# EUROPÄISCHE PATENTSCHRIFT

(11) **EP 0 611 303 B1**
(45) Veröffentlichungstag und Bekanntmachung des Hinweises auf die Patenterteilung: **27.05.1998**
(21) Anmeldenummer: 92921289.2
(22) Anmeldetag: 09.10.1992
(51) Int. Cl.: A61K 31/28, A61K 9/127

(54) **Carboplatin oder Lobaplatin ENTHALTENDE ZUBEREITUNGEN ZUR ANTITUMORTHERAPIE UND/ODER STIMULIERUNG DES HÄMATOPOETISCHEN SYSTEMS**
PREPARATIONS CONTAINING Carboplatin or Lobaplatin FOR ANTI-TUMOUR THERAPY AND/ OR STIMULATING THE HAEMATOPOIETIC SYSTEM
PREPARATIONS RENFERMANT de Carboplatin ou Lobaplatin UTILES EN THERAPEUTIQUE ANTITUMORALE ET/OU POUR LA STIMULATION DU SYSTEME HEMATOPOIETIQUE

(30) Priorität: 11.10.1991 DE 4134158
(43) Veröffentlichungstag der Anmeldung: 24.08.1994
(73) Patentinhaber: MAX-DELBRÜCK-CENTRUM FÜR MOLEKULARE MEDIZIN, D-13125 Berlin (DE)
(72) Erfinder: RESZKA, Regina, D-1297 Schwanebeck (DE); FICHTNER, Iduna, D-1123 Berlin (DE)
(74) Vertreter: Baumbach, Friedrich, Dr.
(86) Internationale Anmeldenummer: DE9200868
(87) Internationale Veröffentlichungsnummer: WO9306824

(56) Entgegenhaltungen:
- EP-A- 0 073 502
- WO-A-90/02131
- WO-A-91/02531
- JOURNAL OF LIPOSOME RESEARCH Bd. 1, Nr. 4, Februar 1991, NEW YORK (US) Seiten 437 - 449 R. PEREZ-SOLER ET AL. 'clinical development of liposomal platinium'
- CHEMICAL ABSTRACTS, vol. 104, no. 18, 5. Mai 1986, Columbus, Ohio, US; abstract no. 155884f, J.B. BASSETT ET AL 'use of temperature-sensitive liposomes in the selective delivery of methotrexate and cis-platinium analogs to murine bladder tumor' Seite 444 ;Spalte 2 ;
- J. MICROENCAPSULATION Bd. 4, Nr. 3, Juli 1987, LONDON (GB) Seiten 201 - 212 R. RESZKA ET AL. 'preparation, characterization, therapeutic efficacy and toxicity of liposomes, containing the antitumour drug cis-dichlorodiamineplatinum (II)'
- CHEMICAL ABSTRACTS, vol. 107, no. 7, 17. August 1987, Columbus, Ohio, US; abstract no. 51471q, D. POPOV ET AL. 'comparative study of the effect of cis- dichlorodiammineplatinum (II) and its aminosugar derivative on hemopoesis in mice' Seite 24-25 ;

## Beschreibung

Die Erfindung betrifft die Verwendung von Carboplatin und Lobaplatin zur Herstellung eines Arzneimittels zur Stimulierung des hämatopoetischen Systems, insbesondere zur Überwindung einer durch Zytostatika ausgelösten Leukopenie oder bei der Behandlung von AIDS.

Liposomen lassen sich als geschlossene, mikroskopische Strukturen charakterisieren, die aus konzentrisch geordneten Amphiphildoppelschichten bestehen, die ihrerseits wässrige Kompartimente voneinander abtrennen.
Wegen ihrer Ähnlichkeit zu Zellmembranen werden Liposomen seit etwa 22 Jahren als multifunktionelles Träger- und Transportsystem für biologisch aktive Substanzen einschließlich pro- und eukariontischer Gene untersucht (Arndt,D., Fichtner, I.(ed.): Liposomen Darstellung - Eigenschaften - Anwendung, Akademie-Verlag, Berlin [1986], Gregoriadis, G. (ed.): Liposomes as drug carriers: Recent trends and progress. John Wiley and Sons. Chichester [1988], Lopez-Berestein, G., Fidler, I. J. (ed.): Liposomes in the therapy of infections diseases and cancer, Alan R. Liss, New York, [1989], Nicolau, C., Cudd, A.: Liposomes as carrier of DNA. Critical Rev. Therapeutic Drug Carrier Systems 6, 239-271 [1989]).
Besonders umfangreich sind die Arbeiten zur liposomalen Verkapselung von Arzneimitteln (Fichtner, I., Arndt, D.: Stand und Perspektiven der Liposomenforschung. Pharmazie **44**, 752-757 [1989]). Liposomen bieten im Vergleich zu anderen Trägersystemen (Nanopartikel, künstliche Zellen usw.) verschiedene Vorteile, die sich unter anderem für die Verkapselung von Zytostatika ausnutzen lassen, wie:
- die Wählbarkeit der Zusammensetzung, Ladung, Größe und Stabilität je nach Fragestellung,
- die Möglichkeit des vollständigen biologischen Abbaus,
- die kaum vorhandenen immunologischen bzw. toxischen Reaktionen,
- die häufig veränderte Pharmakokinetik der liposomal verkapselten Substanz,
- die veränderte Organverteilung und Tropie zu bestimmten Organen,
- die Möglichkeit für verschiedene Methoden des Targetings (Lectine und Antikörper).

Da Liposomen aufgrund ihres amphiphilen Charakters sowohl wasserlösliche als auch lipidlösliche Substanzen einschließen können, sind nahezu alle klinisch etablierten und auch einige in der Entwicklung befindlichen Zytostatika verkapselt und ihre physikochemischen, biochemischen und pharmakologischen Eigenschaften im Vergleich zur jeweiligen Ausgangsverbindung charakterisiert worden (Lit. wie oben). Bei der Anwendung liposomal verkapselter Antineoplastika beobachtet man häufig eine Verminderung der Toxizität bei etwa gleicher therapeutischer Wirksamkeit von liposomaler und freier Form der Substanz.
Überraschend wurde festgestellt daß die Verwendung von Carboplatin oder Lobaplatin vorzugsweise in vesikulärer Form, neben der annähernd gleichen oder wesentlich erhöhten Antitumorwirksamkeit auch/oder zu einer überdurchschnittlichen Stimulierung des hämatopoetischen Systems führt. Die Erfindung wird gemäß Anspruch 1 realisiert. Die Unteransprüche 2 bis 9 sind Vorzugsvarianten.
Erfindungsgemäß wird cis-[(trans-1,2-Cyclobutandimethylamin)-(S)-2-oxidopropanoat-platin (II)] Lobaplatin oder [1,1-Cyclobutandicarboxylat]-platin(II) CBDCA (Carboplatin) - eine Platinkoordinationsverbindung der 2. Generation in lyotrophen Mesophasen wie
- mizellären Systemen
- Mikroemulsionen
- lamellaren Phasen
- hexagonalen Phasen
und oder mit anderen Trägern zur Herstellung eines Arzneimittels zur Stimulierung des hämatopoetischen Systems verwendet.

Die Substanz Carboplatin ist 5-8 fach besser wasserlöslich und besitzt im Vergleich zu cis-Platin durch die Dicarboxylatgruppe
- eine höhere Plasmastabilität,
- eine längere Plasmahalbwertszeit,
- eine geringere irreversible Bindung an Serum- und Plasmaproteine
   und eine schnellere glomeruläre Ausscheidung der verabreichten Platinmenge (Micetich, K.C., Barnes, D., Erickson, L.C.: Cancer Res. **45**, 4043-4047 [1985]).

Reszka et al. (J. Microencapsulation, 1987, Vol 4 (3), p. 201-212) zeigten unter Verwendung von verkapselten cis-DDP ("cis-DDP-REV") eine Stimulierung der Hämatopoese, gemessen als Erhöhung der Leukozytenzahl.

Carboplatin ist wesentlich geringer nierentoxisch als cis-DDP, führt jedoch zu einer ausgeprägten Myelosuppression, die dosislimitierend ist.
Nach Verkapselung von Carboplatin in Liposomen unterschiedlicher Zusammensetzung, Größe, Stabilität und Ladung sowie Applikation äquivalenter Dosen (im Vergleich zur freien Substanz) im Mausmodell, wurde zwar eine geringfügig verminderte therapeutische Aktivität ermittelt, jedoch gleichzeitig eine ausgeprägte Leukozytose beobachtet. Diese mit dem liposomalen Carboplatin erreichte überdurchschnittliche Steigerung der Leukozytenzahlen führte bei Kombination mit Cyclophosphamid zur Verhinderung der durch die Substanz dosislimitierenden Leukopenie. Die Prüfung des Serums der behandelten Tiere an hämatopoetischen Vorläuferzellen (CFU-GM, CFU-G, CFU-M) mit Hilfe der Agarkulturtechnik haben gezeigt, daß es zu einer stark erhöhten Ausschüttung koloniestimulierender Faktoren kommt, die mit GM-CSF oder IL-3 synergistisch wirken können oder selbst eine CSF Wirkung haben (IL-1 bis IL-10, A-Interferon, Tumornekrosefaktor, usw.) Da das natürliche Target der Liposomen Makrophagen/Monozyten sind, lassen sich durch Verkapselung von Carboplatin oder Lobaplatin diese Zellen in einen aktivierten bzw. tumoriciden Zustand versetzen, eine Eigenschaft, die sich für die Entwicklung verschiedener Pharmaka ausnutzen läßt. So ist neben der Stimulierung der Hämatopoese (Behandlung von Aids), die Kombinationschemotherapie (Überwindung der dosislimitierenden Leukopenie klinisch etablierter Zytostatika) auch die physiologische Erzeugung koloniestimulierender Faktoren (eventuell Ablösung der gentechnisch hergestellten, erheblich nebenwirkungsbelasteter Faktoren) möglich.

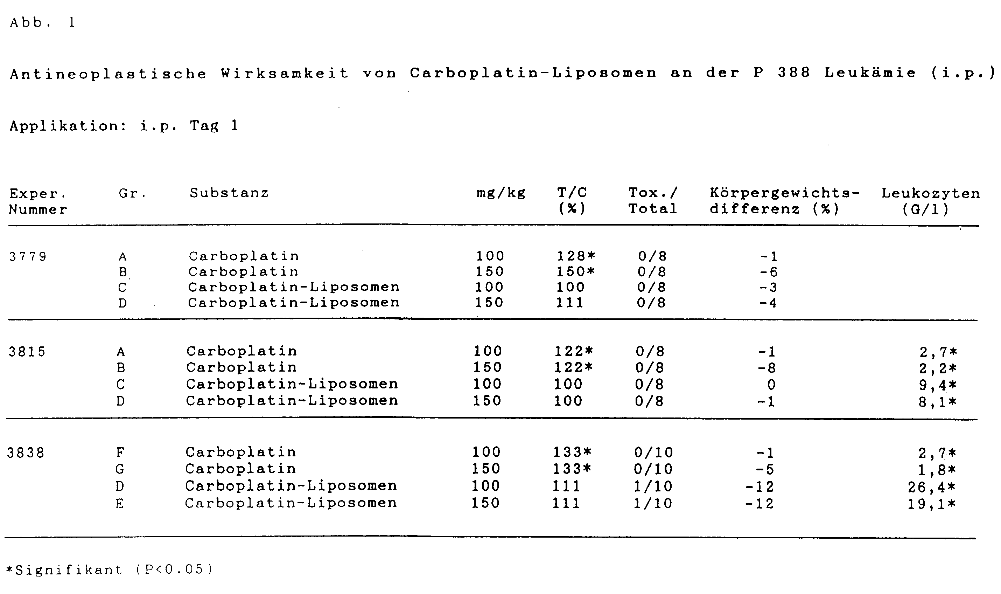

Die durch die erfindungsgemäße Verwendung von liposomalem Carboplatin erhaltenen Ergebnisse werden nachfolgend ausführlicher dargestellt. Getestet wurden Reverse Phase Evaporations Vesikel (REV), da mit Hilfe dieser Liposomen die für tierexperimentelle Untersuchungen notwendige Substanzmenge eingeschlossen werden konnte. Die Charakterisierung der Vesikel hinsichtlich ihrer Größe und Lamellarität erfolgte mit der Elektronenmikroskopie (Negativkontrast) sowie in ausgewählten Fällen zur Bestimmung der Größenverteilung mit der quasielastischen Lichtstreuung. Die Bestimmung der Einschlußraten wurde mit einer indirekten Methode, der kolloidalen Bindung an Zinnchlorid, durchgeführt. In wiederholt durchgeführten Tierexperimenten an der P 388 Leukämie der Maus (i.p.) wurden, gemessen am therapeutischen Indem (%T/C) und an den Toxizitätsparametern (Körpergewichtsdifferenz und Leukozytenzahlen) im Vergleich zur Kontrollgruppe (mit physiologischer Kochsalzlösung behandelte Tiere), nach i.p. Applikation von 100 bzw. 150 mg Carboplatin in freier bzw. liposomaler Form folgende Ergebnisse erhalten:
- Verminderung des ohnehin nicht überragenden zytostatischen Effekts von Carboplatin an der P 388 nach liposomaler Verkapselung.
- Signifikante Erhöhung der Leukozytenzahlen nach Gabe der liposomalen Form von Carboplatin, während die freie Form einen leukopenischen Effekt zeigt (Abb.1).

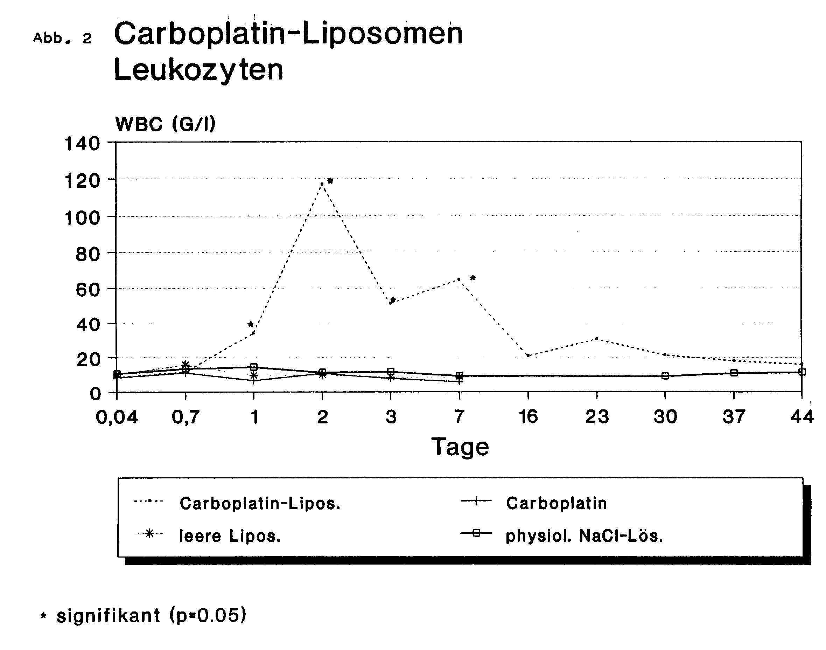

Zur weiterer Charakterisierung des Leukozyteneffektes wurden Carboplatin-haltige REV, freies Carboplatin (100 mg/kg), leere REV und physiologische Kochsalzlösung an tumorfreie Tiere appliziert und an verschiedenen Tagen Blut abgenommen. Hier zeigte sich, daß mit dem liposomal verkapselten Carboplatin am 2. Tag und am 7. Tag ein Leukozytengipfel auftrat, der mit 120.000 bzw. 60.000 G/l das 10- bzw. 5fache des Normalwertes betrug (Abb. 2).

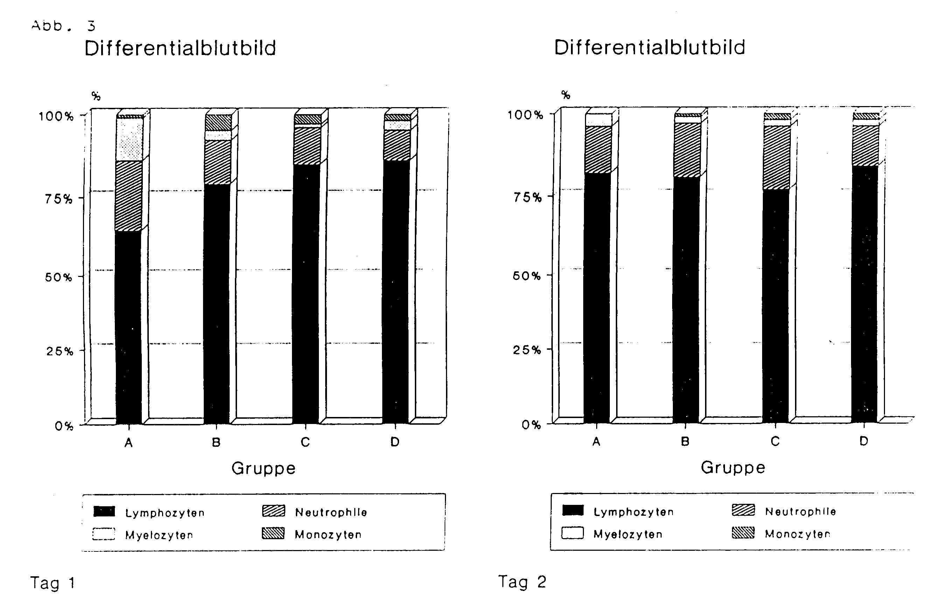

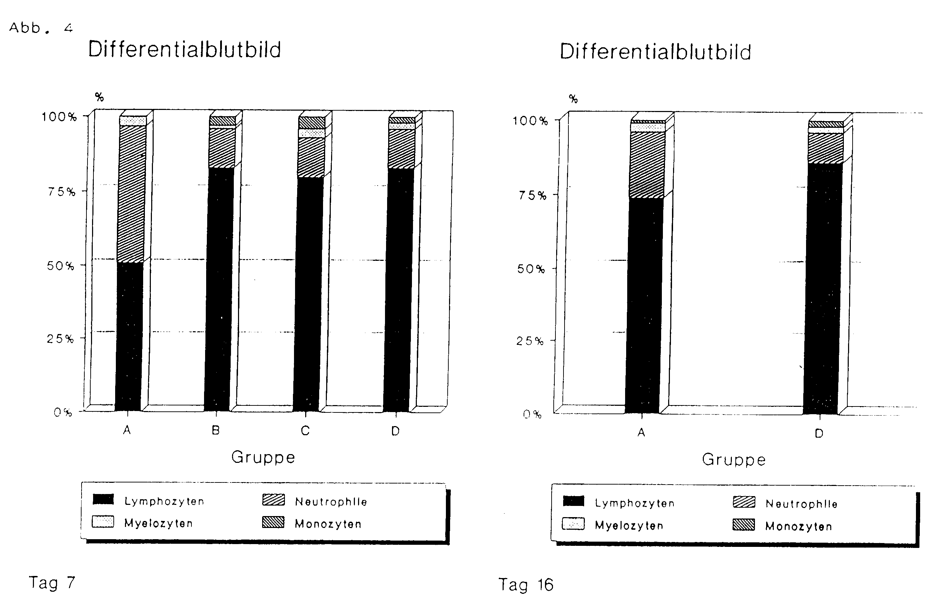

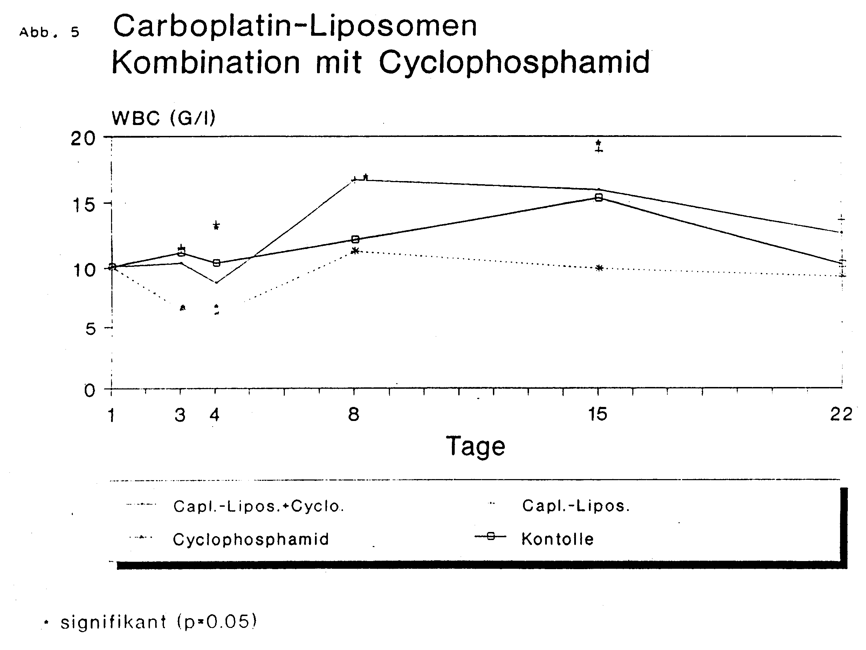

Untersuchungen dieser Leukozytenpopulation im Differenzialblutbild ergab am 1. Tag eine relative Zunahme der Neutrophilen und Myelozyten, was eine Linksverschiebung in der Haematopoese anzeigt (Abb. 3). Am 7. Tag waren 50 % Lymphozyten und 45 % Neutrophile nachweisbar, eine Tendenz, die auch noch am 16. Tag vorhanden war (Abb. 4). Der 1. Gipfel deutet auf eine Ausschüttung von Leukozyten aus den peripheren Speichern hin, während der 2. Gipfel für eine Ausschüttung von Leukozyten aus dem Knochenmark spricht. Dieser Effekt läßt sich klinisch ausnutzen, um z.B. die Leukopenie myelosupressiver Zytostatika zu verringern bzw. zu verhindern, was durch eine Kombination mit Cyclophosphamid belegt wurde. Eine Stunde vor Applikation von 100 mg/kg Cyclophosphamid wurden Carboplatin-Liposomen (50 mg/kg) verabfolgt. Während Cyclophosphamid alleine zu einer signifikant ausgeprägten Leukopenie führt, Carboplatin-Liposomen alleine eine Leukozytose auslösen, führt die Kombination zur Erreichung der Leukozyten-Normalwerte gemessen an der Kontrollgruppe (Abb. 5).

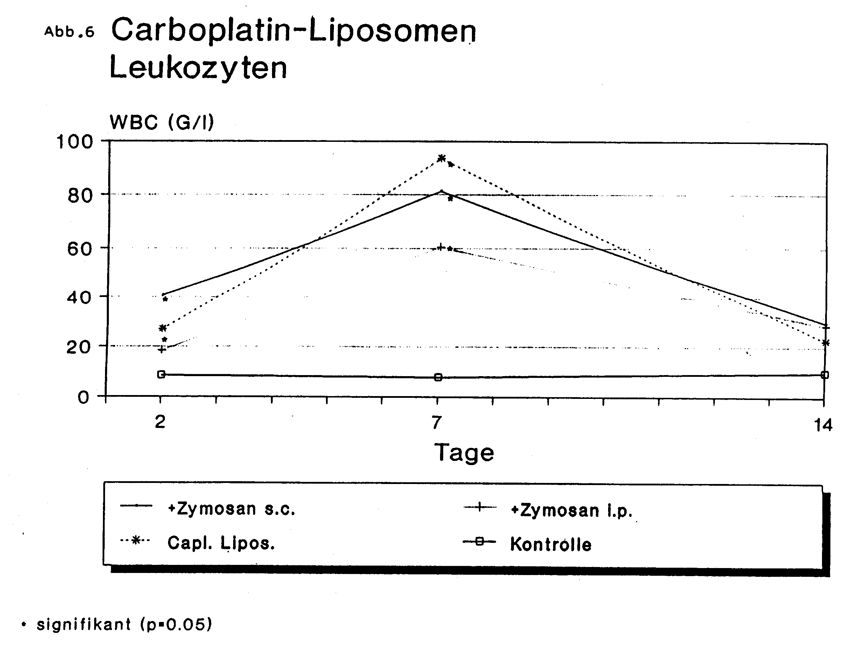

Diese Ergebnisse lassen sich folgendermaßen interpretieren:
Potentielles Target der Liposomen sind die Makrophagen. Um ihren Einfluß zu prüfen, wurden Mäuse einen Tag vor Liposomengabe mit 100 mg/kg Zymosan (Makrophagen-blockierende Substanz) s.c. bzw. i.p. behandelt. Carboplatin-Liposomen ohne Vorbehandlung zeigten die bereits beschriebene Leukozytenstimulation. Die Gabe von Zymosan s.c. vor dem liposomal verkapselten Präparat beeinflußte diese Wirkung nicht. Bei Applikation des Zymosans i.p. war eine deutliche, wenn auch nicht vollständige Verminderung der Leukozytenstimulation nachweisbar (Abb. 6). Deshalb ist davon auszugehen, daß die Makrophagen/Monozyten als natürliches Target der Liposomen bzw. die von ihnen ausgeschütteten Zytokine (Granolzyten-Monozyten-Aktivierungsfaktoren; Interleukine, z.B. IL 3, IL 6) maßgeblich für den haematopoetischen Effekt verantwortlich sind. Gestützt werden diese Befunde durch erste Versuche zur Charakterisierung der koloniestimulierenden Aktivität im Knochenmarkstammzelltest. Das Serum behandelter Tiere zeigte nach i.p. Applikation des liposomal verkapselten Carboplatins eine 12fache Stimulation des Koloniewachstums. Liposomales Carboplatin kann damit zu einem endogenen Stimulator der Haematopoese werden, was in der kombinierten Anwendung bei der Strahlen- bzw. Chemotherapie sowie bei der Aidstherapie verbesserte Effekte erwarten läßt.
Auch der Einsatz von liposomalem Lobaplatin {cis[(trans-1,2-Cyclobutandimethylamin)-(S)-2-oxidopropanoat-platin (II)]} führt gemäß der Erfindung zu einer erheblichen Steigerung der Leukozytenzahlen. Versuche an Lewis Lung-Tumoren (i.v.) zeigten nach einer Therapie i.p. am 1. Tag eine ca. 3fache Steigerung von 9,2 auf 33,4 G/l. Die Bestimmung der Leukozytenzahlen wurde dabei am 4. Tag vorgenommen.
Die Erfindung soll nachfolgend an Ausführungsbeispielen näher erläutert werden.

### Ausführungsbeispiele

### Beispiel 1

Aus einer Mischung von 2328 Eiphosphatidylcholin (Herstellung entsprechend Singleton et al., J. Am. Oil Chemist's Soc. **42**, 53-56 [1965]) und 1132 mg Cholesterol (entspr. USP XVIII), wird ein Lipidfilm hergestellt, den man mit einem Gemisch, bestehend aus 450 ml Tetrahydrofuran und 60 ml steriler, Calcium-freier, Phosphat-gepufferter (pH 7,2 - 7,4) Kochsalzlösung, die 900 mg Carboplatin enthält, dispergiert. Anschließend wird das organische Lösungsmittel am Rotationsverdampfer, bei unterschiedlichen Vakuumstufen abdestilliert und die sich über eine Gelzwischenphase bildende Liposomendispersion zur Abtrennung des nicht verkapselten Wirkstoffs bei 40 000 U/min., 1 h, zentrifugiert (3* mit je der 10fachen Puffermenge im Überschuß, bei 4 Grad Celcius). Nachdem das Pellet (im Anschluß an die letzte Zentrifugation) in der gewünschten Menge Puffer resuspendiert ist, extrudiert man die Liposomenlösung über Filtermembranen (2,0; 1,0; 0,8; 0,4 und 0,2 µm). Die erhaltene Vesikelsuspension ist bei 4 Grad Celcius lagerfähig und eignet sich zur parenteralen (i.v.) Applikation.

### Beispiel 2

Aus einer Mischung von 2328 mg hydriertem Eiphosphatidylcholin (Herstellung entsprechend Reszka et al. , Pharmazie **44**, 503 [1989]) und 1132 mg Cholesterol (entspr. USP XVIII) werden mit denen in Beispiel 1 beschriebenen Zusätzen in gleicher Weise Liposomen hergestellt und ebenso wie dort weiterbehandelt. Die erhaltene Liposomendispersion ist bei 4 Grad Celcius lagerfähig und eignet sich zur parenteralen (i.v.) Applikation.

### Beispiel 3

Einen Lipidfilm, bestehend aus 1164 mg hydriertem Eiphosphatidylcholin (Herstellung Reszka et al., Pharmazie **44**, 503 [1989]) dispergiert man in 225 ml Tetrahydrofuran und 30 ml, steriler, Calcium-freier, Phosphat-gepufferter (pH 7,2 - 7,4) Kochsalzlösung und behandelt diese Suspension in gleicher Weise weiter, wie in Beispiel 1 beschrieben. Die erhaltene Liposomendispersion ist bei 4 Grad Celcius lagerfähig und eignet sich zur parenteralen (i.v.) Applikation.

### Beispiel 4

Ein Lipidfilm, bestehend aus 150 mg Eiphosphatidylcholin (Herstellung entsprechend Singleton et al., J. Am. Oil Chemist's Soc. **42**, 53-56 [1965]), wird in 3,86 ml steriler, Calciumfreier, Phosphat-gepufferter (pH 7,2 - 7,4) Kochsalzlösung, die 19,35 mg Carboplatin enthält, dispergiert. Die entstandene Dispersion multischichtiger Liposomen kann nun in schon beschriebener Weise durch Filtermembranen extrudiert werden. Die erhaltene Liposomendispersion ist bei 4 Grad Celcius lagerfähig und eignet sich zur parenteralen (i.v.) Applikation.

### Beispiel 5

Ein Lipidfilm, bestehend aus 150 mg hydriertem Eiphosphatidylcholin (Herstellung entsprechend Reszka et al., Pharmazie **44**, 503 [1989]), wird in 3,86 ml steriler, Calciumfreier, Phosphat-gepufferter (pH 7,2-7,4) Kochsalzlösung, die 19,35 mg Carboplatin enthält, dispergiert. Die entstandene multischichtige Vesikeldispersion wird nun, wie in Beispiel 4 beschrieben, weiterbehandelt und eignet sich bei einer Lagerungstemperatur von 4 Grad Celcius zur parenteralen (i.v.) Applikation.

### Beispiel 6

Aus einer Mischung von 129,2 mg hydriertem Eiphosphatidylcholin (Herstellung entsprechend Reszka et al., Pharmazie **44**, 503 [1989]), 31,13 mg Cholesterol (entspr. USP XVIII) und 9,04 mg Dicethylphosphat (Dihexadecylhydrogenphosphat), reinst, (Serva) wird ein Lipidfilm hergestellt, der in einem Gemisch, bestehend aus 25 ml Tetrahydrofuran und 2,33 ml steriler, Calcium-freier, Phosphat-gepufferter (pH 7,2-7,4) Kochsalzlösung, in der 50 mg Carboplatin gelöst wurden, dispergiert. Anschließend wird die Suspension, wie im Beispiel 1 beschrieben, weiterbehandelt. Es entsteht eine Liposomendispersion, die bei 4 Grad Celcius lagerfähig und zur parenteralen (i.v.) Applikation geeignet ist.

### Beispiel 10

Aus einer Mischung von 517 mg hydriertem Eiphosphatidylcholin (Herstellung entsprechend Reszka et al., Pharmazie **44**, 503 [1989]) und 215 mg Cholesterol (entspr. USP XVIII) wird ein Lipidfilm hergestellt, der in einem Gemisch aus 100 ml Tetrahydrofuran und 13,3 ml steriler, Calcium-freier, Phosphat-gepufferter (pH 7,2-7,4) Saccharose-haltiger (338 mmol/l) Kochsalzlösung, die 200 mg Carboplatin enthält, dispergiert wird. Die Suspension wird in gleicher Weise weiterbehandelt, wie in Beispiel 1 beschrieben. Die Liposomendispersion wird anschließend in einer Gefriertrockenanlage lyophilisiert und unter Schutzgas verschlossen. Vor Gebrauch werden zu diesem Trockenpräparat unter sterilen Bedingungen 13,3 ml destilliertes Wasser (steril, pyrogenfrei) gegeben und das Gefäß auf einem Vortex-Mixer zehn Minuten lang geschüttelt. Die resultierende Liposomendispersion eignet sich zur parenteralen (i.v.) Applikation.

### Beispiele 11-16, 20

Wie Beispiele 1-6, 10, nur unter dem Einsatz von 900 mg Lobaplatin anstelle von Carboplatin.

## Patentansprüche

1. Verwendung von Carboplatin bzw. Lobaplatin in lyotrophen Mesophasen, wie
- mizellären Systemen,
- Mikroemulsionen,
- lamellaren Phasen,
- hexagonalen Phasen
und/oder mit anderen Trägern zur Herstellung eines Arzneimittels zur Stimulierung des hämatopoetischen Systems.

2. Verwendung nach Anspruch 1 zur Herstellung eines Arzneimittels zur Überwindung einer durch Zytostatika ausgelösten Leukopenie.

3. Verwendung nach Anspruch 1 zur Herstellung eines Arzneimittels zur Stimulierung des hämatopoetischen Systems bei der Behandlung von AIDS.

4. Verwendung nach Anspruch 1 bis 3, dadurch gekennzeichnet, daß die Arzneimittel in vesikulärer Form vorliegen.

5. Verwendung nach Anspruch 1 bis 3, dadurch gekennzeichnet, daß die Arzneimittel in liposomaler Form vorliegen.

6. Verwendung nach Anspruch 1 bis 5, dadurch gekennzeichnet, daß die Arzneimittel
a) ein natürliches, halbsynthetisches oder vollsynthetisches Amphiphil der allgemeinen Formel I 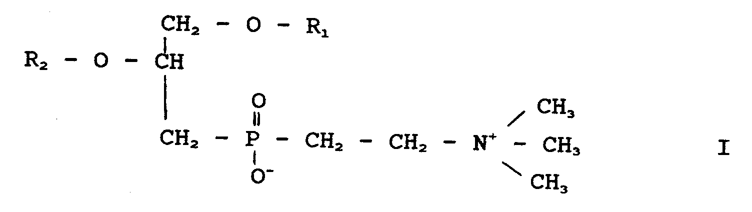 worin R₁ und R₂ = C₁₀ - C₂₀ -Alkanoyl, -Alkenoyl, -Alkyl, -Alkenyl bedeuten,
b) ein Steroid,
c) das Anion des Diacethylphosphats, der Palmitinsäure, der Stearinsäure, das Anion eines Phospholipids, wie Phosphatidylserin, Phosphatidsäure, oder das Anion eines Sphingolipids, wie Sulfatid,
d) Carboplatin bzw. Lobaplatin
e) eine Trägerflüssigkeit
f)gegebenenfalls zusätzliche Hilfsstoffe, wie Nanopartikel enthalten.

7. Verwendung nach Anspruch 6, dadurch gekennzeichnet, daß die Arzneimittel
b) ein Steroid der allgemeinen Formel II 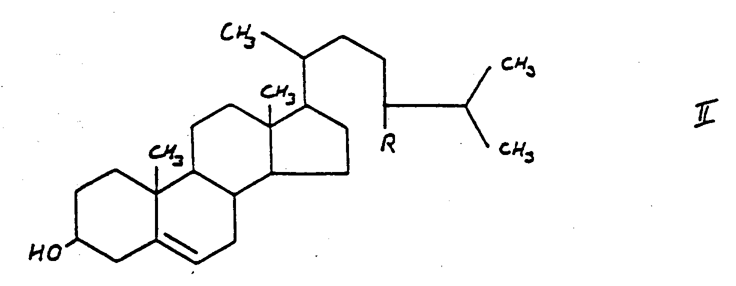 in der R = H = Cholesterol
oder R = CH₂-CH₂-O-CH₂-CH₂-OH = Diethoxycholesterol bedeutet,
enthalten.

8. Verwendung nach Anspruch 6 und 7, dadurch gekennzeichnet, daß die Komponenten a:b:c im Molverhältnis
1:0,3:0,1 bis 1:1:0,1 oder bis 1:1:0,5 vorliegen.

9. Verwendung nach Anspruch 6 und 7, dadurch gekennzeichnet, daß die Komponenten c:d im Molverhältnis zwischen
2:1 bis 10:1 vorliegen.

## Claims

1. Use of carboplatinum or lobaplatinum in lyotrophic mesophases such as
- micellular systems,
- microemulsions,
- lamellar phases,
- hexagonal phases
and/or with other carriers to produce a pharmaceutical agent for the stimulation of the haematopoietic system.

2. Use according to claim 1 for the production of a pharmaceutical agent to overcome a leucopenia caused by cytostatic agents.

3. Use according to claim 1 for the production of a pharmaceutical agent to stimulate the haematopoietic system when treating AIDS.

4. Use according to claims 1 to 3 marked by the fact the pharmaceutical agents are available in a vesicular form.

5. Use according to claims 1 to 3 marked by the fact that the pharmaceutical agents are available in a liposomal form.

6. Use according to claims 1 to 5 marked by the fact that the pharmaceutical agents contain
a) mean a natural, semisynthetic or fully synthetic amphilile of the general formula I 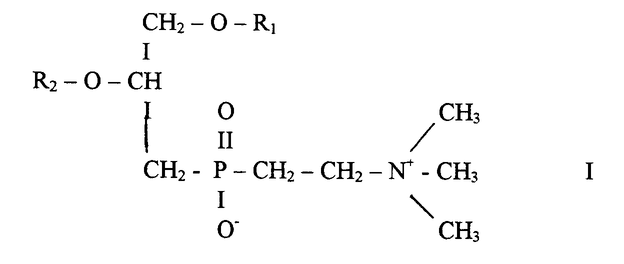 with R₁ and R₂ = C₁₀ - C₂₀ - alkanoyl, -alkenoyl, - alkyl, -alkenyl,
b) a steroid,
c) the anion of biacethyl phosphate, palmitic acid, stearic acid, the anion of a phospholipid such as phosphatidyl serine, phosphatidic acid, or the anion of a sphingolipid such as sulphatide,
d) carboplatinum or lobaplatinum
e) a carrier liquid
f) in the case of need, additional auxiliary agents such as nano particles

7. Use according to claim 6 marked by the fact that the pharmaceutical agents contain
b) a steroid of the general formula II 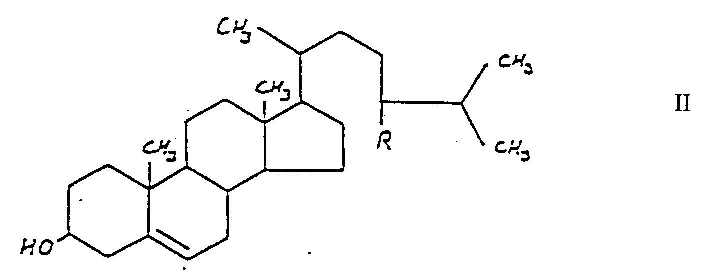 with R = H cholesterol
or R = CH₂- CH₂ - O - CH₂ - CH₂ - OH = diethoxycholesterol.

8. Use according to claims 6 and 7 marked by the fact that the components are available in the molar ratio
1:0, 3:0.1 up to 1:1:0.1 or up to 1:1:0.5.

9. Use according to claims 6 and 7 marked by the fact that the components c:d are available in the molar ratio between
2:1 and 10:1

## Revendications

1. Utilisation de carboplatine et/ou lobaplatine dans des mésophases lyotrophes telles que
- systèmes micellaires
- microémulsions
- phases lamellaires
- phases hexagonales
et/ou avec d'autres supports pour la préparation d'un médicament destiné à stimuler le système hématopoëtique.

2. Utilisation selon la revendication 1 d'un médicament destiné à supprimer une leucopénie due à des agents cytostatiques.

3. Utilisation selon la revendication 1 pour la préparation d'un médicament destiné à stimuler le système hématopoëtique pendant le traitement du sida.

4. Utilisation selon les revendications 1 à 3, caractérisée en ce que le médicament se présente sous forme vésiculaire.

5. Utilisation selon les revendications 1 à 3, caractérisée en ce que le médicament se présente en forme de liposomes.

6. Utilisation selon les revendications 1 à 5, caractérisée en ce que les médicaments contiennent
a) une molécule amphiphile naturelle, semisynthétique ou entièrement synthétique de formule générale I 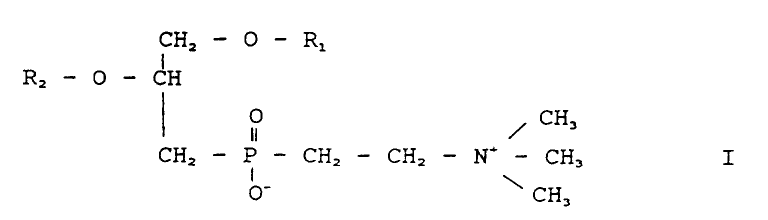 où signifient R₁ et R₂ = C₁₀ - C₂₀ -alcanoyle, -alcénoyle, -alkyle, -alcényle,
b) un stéroïde,
c) l'anion du diacétylphosphate, de l'acide palmitique, de l'acide stéarique, l'anion d'un phospholipide tel que phosphatidylsérine, acide phosphatidique, ou l'anion d'un sphingolipide tel que sulfatide,
d) carboplatine et/ou lobaplatine,
e) un support liquide,
f) le cas échéant, des matières auxiliaires complémentaires telles que nanoparticules.

7. Utilisation selon la revendication 6, caractérisée en ce que les médicaments contiennent
b) un stéroïde de formule générale II 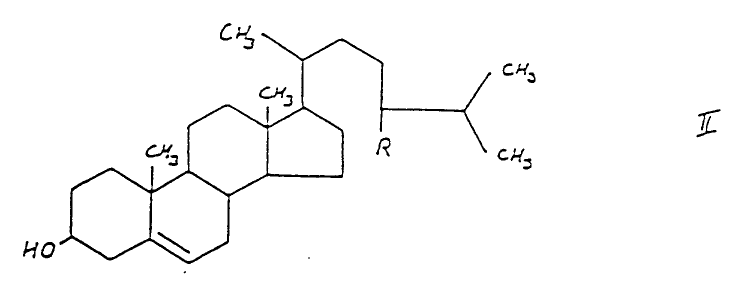 où signifie
R = H = cholestérol
ou R = CH₂-CH₂-O-CH₂-CH₂-OH = diéthoxycholestérol.

8. Utilisation selon les revendications 6 et 7, caractérisée en ce que les composantes a:b:c se présentent dans un rapport molaire
allant de 1:0,3:0,1 à 1:1:0,1 ou à 1:1:0,5.

9. Utilisation selon les revendications 6 et 7, caractérisée en ce que les composantes b:c se présentent dans un rapport molaire compris entre
2:1 et 10:1.
